Europäisches Patentamt

European Patent Office

Office européen des brevets

(11) Publication number: **0 408 346 A1**

# EUROPEAN PATENT APPLICATION

(21) Application number: 90307599.2

(51) Int. Cl.⁵: **A61K 33/30, A61K 9/72**

(22) Date of filing: **11.07.90**

(30) Priority: **12.07.89 US 379470**
**22.06.90 US 534121**

(43) Date of publication of application:
**16.01.91 Bulletin 91/03**

(84) Designated Contracting States:
**AT BE CH DE DK ES FR GB GR IT LI LU NL SE**

(71) Applicant: **Sabin, Robert**
**Goosedown Estate, Horseshoe Road, Mill Neck**
**Long Island, New York 11765(US)**

(72) Inventor: **Sabin, Robert**
**Goosedown Estate, Horseshoe Road, Mill Neck**
**Long Island, New York 11765(US)**

(74) Representative: **Allard, Susan Joyce et al**
**BOULT, WADE & TENNANT, 27 Furnival Streeteet**
**London EC4A 1PQ(GB)**

(54) **Therapeutic agent for stimulating the immune system.**

(57) The invention provides for use of zinc, zinc oxide, zinc alloy or other compound which yields zinc oxide on heating, in the preparation of a therapeutic agent for use in the treatment of cancer and/or infection. The therapeutic agent when inhaled stimulates and induces a physical immune response for the treatment of cancer or infection, including viral, bacterial or fungal infection by inducing a condition manifested by fever, profuse perspiring, polymorphonuclear leukocytosis and shaking.

EP 0 408 346 A1

## THERAPEUTIC AGENT FOR STIMULATING THE IMMUNE SYSTEM

The present invention is directed to use of zinc containing substances in the preparation of a therapeutic agent for treatment of cancer and/or infections.

The therapeutic agent may be used in a method for actively and passively stimulating the immune system by inhalation of fumes of the agent which is produced from melting and fuming of, for example, copper-zinc metal alloys or zinc metal.

Passive immunotherapy refers to approaches in which immunologic reagents, such as serum, cells, or cell products that are thought to have antitumor activity are administered to a tumor-bearing host. Active immunotherapy refers to situations in which the host is stimulated to produce an immune response that directly or indirectly causes tumor-cell death.

For hundreds of years physicians have attempted to induce artificial fever to combat disease since at least the time of Parmenides. A fever is a manifestation of an extreme response by the body's immune system to combat infections. Over the last two hundred years, work has focused around the observation of both spontaneous regression of cancer, and cancer regression, with simultaneous bacterial infections. Thus, erysipelas infections, staphylococcal infection, and pyogenic infections were utilized to treat cancer patients to produce permanent regressions and remiss ions. More recently, Coley's mixed toxins (bacterial toxins), interleukin-2 and interferons have been administered to stimulate the immune system to combat cancer, since all forms of cancer including carcinoma and melanoma, represent abnormal cells in the body which, for many possible reasons, have not been contained by the body's immune system. Moreover, over three hundred true spontaneous regressions of cancer have been reported.

Historically, cancer medicine has been administered through, and in combination with, the three standard regimens of surgery, radiation, and chemotherapy. Presently, biotherapy - the administration of biological and biological response modifiers to shock, jolt, amplify, potentiate and commandeer the body's own immune system to fight cancer is the fourth modality. Biological response modifiers are defined as those agents or approaches that modify the relationship between the tumor and host by modifying the host's biological response to tumor cells with resultant therapeutic effects. Biologicals, and biological response modifiers under investigation, development of utilization include:

1. Interferons
2. Interleukins
3. Colony stimulating factor
4. Monoclonal antibodies
5. Differentiating agents
6. Anti-Metastastis agents
7. Thymosins
8. Coley's mixed toxins
9. Bacterial agents - i . e ., B.C.G. and Corynebacterium parvum
10. Vitamin A
11. Vitamin C
12. Cimetidine
13. Levamisole
14. Lymphokines
15. Cytokines
16. Tumor Necrosis Factor

It is thus an object of the present invention to provide a therapeutic agent for use in the fourth modality of biotherapy, for stimulating the immune system and inducing polymorphonuclear leukocytosis and artificial fever, to thereby stimulate the body to an extreme immune response, and to thus combat cancer and all types of infection, including viral, fungal or bacterial infection, particularly in the early stages when there is a higher likelihood that the cancer or infection will be responsive to treatment.

In accordance with the invention zinc, zinc oxide, zinc alloy or other zinc compound which yields zinc oxide on heating are used in the preparation of a therapeutic agent for use in the treatment of cancer and/or infection.

The use of the aforementioned zinc containing substances in such a manner provides a method for the treatment of cancer or infection, including viral, fungal or bacterial infection. The method comprises the step of exposing a subject afflicted with or suspected of being afflicted with cancer or infection, to a low concentration of fumes from fuming for example zinc or copper-zinc alloy, for inhalation over a period of time sufficient to induce the condition known as "brass founders ague", "zinc shakes", "brass chills", "shakes", "zinc metal fume fever", "galvo", "zinc chills", "foundry ague", "foundry shakes", "Monday morning fever", "spelten shakes" or "Brazier's disease" manifested by fever in the subject within about eight hours of such exposure, which condition dissipates within one to two days after such exposure.

A subject afflicted with cancer or infection of any type or suspected of being afflicted with cancer or infection of any type may be treated by the above described method and is treated by inhalation of small quantities of fumes from fuming zinc or copper-zinc alloy. The fume is produced by heating above its melting point, preferbaly, a copper zinc alloy, although any zinc alloy, pure zinc metal or other zinc compound may be utilized to produce a freshly formed fume which comprises zinc oxide. It is preferred that a 70% copper, 30% zinc alloy by weight be utilized since copper does not produce a metal vapor or metal oxide vapor at the temperatures at which the zinc metal fumes to form oxide. Furthermore, CuZn alloys are preferred since alloys have melting points (598°C for 88% Zn, balance Cu; 834°C for 58% Zn, balance Cu; 902°C for 37% Zn, balance Cu; 419°C for pure Zn, and 1083°C for pure Cu) attainable by a common oil or gas fired furnace crucible. The crucible may be preferably made of alumina, or a hardened silicon carbide. Preferably an open silicon carbide crucible will be used so as to expose the molten metal to the atmosphere. The quantity of fumes is regulated by modulating the temperature of the crucible by adjusting the oil, gas or voltage to maintain the temperature at, below or above a point Zn begins to fume off from the molten bath of metal in the crucible. An electric crucible furnace may also be used.

To ensure that the subject is exposed only to a small concentration of the zinc oxide fumes, the treatment should be conducted in an enclosed space, such as a normal-sized room ( i . e ., 9 to 20 feet per side with normal ceiling height) with normal ventilation, such as by ventilation ducting or open windows, to provide sufficient ventilation to avoid the possibility of carbon monoxide poisoning from the combustion heater, without overventilation which may exhaust the zinc oxide too rapidly from the room. The zinc oxide fumes form a white fume.

Typically, about 40 lbs. of copper zinc alloy (preferably 63% Cu, 37% Zn) will be melted a the crucible in a room and heated to a temperature of at least about 1800° F to cause the zinc to fume to produce a freshly formed zinc oxide fume. A fine zinc dust or zinc oxide dust may alternatively be used, but are less preferred. It will normally be sufficient for the subject to be in the room, at rest, for up to about 1 hour after the zinc begins to fume. With persons of normal body weight the period of exposure to the fume should be sufficient to induce a fever, polymorphonuclear leukocytosis and profuse perspiring in the subject within about 2-8 hours subsequent to exposure. Although the exposure to the zinc oxide may exceed an hour in extreme cases, it is sufficient that such exposure be less than an hour. The quantity of fumes may be modulated by adjustment of the temperature of the furnace.

The preferred dosage, in terms of airborne level of zinc oxide fume concentration in the inhaled air, is from about 15 mg/m$^3$ to about 900 mg/m$^3$ for up to about 1 hour, at rest. Measurement of fume concentration may be accomplished according to procedures disclosed in NIOSH J. of Analytical Methods , 2nd ed., Vol. 4 , 1978, GPO#017-033-00317-3.

Continual exposure to fumes of zinc oxide may engender tolerance thereto, however, this tolerance may be nullified by intermittent exposure, or cessation of exposure for several days. Furthermore, since Vitamin C has been shown to potentiate the immune system, it is preferred that Vitamin C also be co-administered in a dosage range of 1-30 grams/adult subject prior to or simultaneous with exposure to the fumes. Simultaneous co-administration of Vitamin A in the amount of 50,000 to 1x10$^6$ I.U./adult subject also has a potentiating effect.

Induction of the condition is accompanied by shaking, chills, polymorphonuclear leukocytosis and profuse perspiring, commonly known by the names "zinc shakes", etc., mentioned above. Treatment is preferably repeated approximately every 4 to 10 days. There is no known cumulative effect to inhalation of the freshly formed fumes. The physical manifestations of the "zinc shakes" disappear within about 24-48 hours.

Since the zinc oxide is a common additive to pharmaceutical carriers, it is not believed that inhalation of zinc oxide is harmful to the subject, particularly in the dosages prescribed under the above-described treatment.

Other methods of inhalation may be utilized such as by control of zinc oxide vapor through an inhalation mask, mixed with oxygen. However it is realized that such treatment would need to be carefully controlled to ensure the proper concentration of breathable air and zinc oxide within the prescribed amounts.

## Claims

1. Use of zinc, zinc oxide, zinc alloy or other zinc compound which yields zinc oxide on heating, in the preparation of a therapeutic agent for use in the treatment of cancer and/or infection.

2. The use as claimed in claim 1 wherein said therapeutic agent is gaseous zinc oxide.

3. The use as claimed in claim 2 wherein the preparation of said gaseous zinc oxide is by heating the said zinc, zinc oxide, zinc alloy or other zinc compound in a furnace crucible.

4. The use as claimed in claim 3 wherein said crucible is a common oil or gas fired furnace crucible or an electric furnace crucible.

5. The use as claimed in claim 3 or claim 4 wherein said crucible is made from alumina or more preferably hardened silicon carbide.

6. The use as claimed in any preceding claim wherein zinc is a starting material in the preparation of said therapeutic agent.

7. The use as claimed in any one of claims 1 to 5 wherein zinc oxide dust is a starting material in the preparation of said therapeutic agent.

8. The use as claimed in any one of claims 1 to 5 wherein copper/zinc alloy is a starting material in the preparation of said therapeutic agent.

9. The use as claimed in claim 8 wherein said copper/zinc alloy is 70% copper and 30% zinc.

10. A process for the preparation of a medicament for treating cancer and/or infection which comprises heating zinc, zinc oxide, zinc alloy or other zinc compound to produce gaseous zinc oxide.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 291 164 (SPECTRAL BIOANALYSIS LTD) <br> * Page 7, line 43 (claim 10) * <br> --- | 1-10 | A 61 K 33/30 <br> A 61 K 9/72 |
| X | CHEMICAL ABSTRACTS, vol. 110, no. 9, 27th February 1989, page 208, abstract no. 70864y, Columbus, Ohio, US; K.S. KASPRZAK et al.: "Inhibitory effect of zinc on nickel subsulfide carcinogenesis in Fischer rats", & TOXICOLOGY 1988, 52(3), 253-62 <br> * Abstract * <br> ----- | 1-10 | |

TECHNICAL FIELDS
SEARCHED (Int. Cl.5)

A 61 K

The present search report has been drawn up for all claims

| Place of search | Date of completion of the search | Examiner |
|---|---|---|
| THE HAGUE | 01-10-1990 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)